# EUROPÄISCHE PATENTSCHRIFT

(11) **EP 2 926 839 B1**
(45) Veröffentlichungstag und Bekanntmachung des Hinweises auf die Patenterteilung: **12.06.2019**
(21) Anmeldenummer: 15162677.7
(22) Anmeldetag: 07.04.2015
(51) Int. Cl.: A61L 15/18, A61L 15/44, A61K 9/00, A61K 33/08, A61K 33/14, A61K 33/42

(54) **HAUTAUFLAGE**
SKIN PATCH
TIMBRE TRANSDERMIQUE

(30) Priorität: 03.04.2014 CH 5172014
(43) Veröffentlichungstag der Anmeldung: 07.10.2015
(73) Patentinhaber: Stiftung für Plastische und Aesthetische Wundheilung im Sondervermögen der DT Deutschen Stiftungstreuhand AG, 90762 Fürth (DE)
(72) Erfinder: Gehl, Dr., Gerolf, 9306 Freidorf Thurgau (CH)
(74) Vertreter: Rentsch Partner AG

(56) Entgegenhaltungen:
- WO-A2-2009/128076
- CH-A2- 705 253
- US-A1- 2010 228 174

## Beschreibung

### TECHNISCHES GEBIET

Die vorliegende Erfindung betrifft eine Hautauflage gemäss dem Oberbegriff des unabhängigen Patentanspruchs 1.

### STAND DER TECHNIK

Vom Anmelder ist aus der EP 1 339 417 B1 und CH 705 253 A2 eine Zubereitung zur Behandlung von flächigen, offenen Wunden bekannt, welche eine feste aktive Komponente aufweist, die ein Salz eines biologisch abbaubaren Phosphates sowie Calzium- und Alkalimetallionen enthält. Diese Zubereitung hat sich bei der Behandlung von Wunden an Oberflächengeweben, die beispielsweise durch einen Dekubitus, Verbrennungen, UV-Strahlen oder Chemikalien hervorgerufen sind, hervorragend bewährt.

Die genannte Zubereitung gemäss der EP 1 339 417 B1 hat den wesentlichen Vorteil, dass sie das behandelte Gewebe zum Wachstum anregt, so dass durch das neue Gewebe nicht nur ein Defekt verschlossen, sondern auch ein Aufbau einer Gewebestruktur angeregt werden kann. Diese Wirkung der Zubereitung - auch Substanz genannt - ist in der WO 09121616 A des Anmelders offenbart, deren Offenbarung hiermit ebenfalls vollumfänglich mit einbezogen wird.

Die bekannten Zubereitungen werden direkt auf die Wunde aufgebracht oder aber auf einem - vorzugsweise festen - Träger appliziert. Mögliche Träger sind Wachs, Wachsfolien, Wachsschablonen oder Siliconträger. Es ist bereits beschrieben, dass kraterförmige Vertiefungen der Wunden mit den bekannten Zubereitungen ausgefüllt und eingeebnet werden.

Trotz der beeindruckenden therapeutischen Erfolge bei der Wundheilung besteht das Bedürfnis die Anwendung der Zusammensetzungen weiter zu erleichtern und die Anwendungsbereiche der bekannten Zubereitungen zu vermehren.

Eine Hautauflage mit bioaktivem Glas ist bekannt: US2010/0228174 A1.

Zudem besteht ein anhaltendes Bedürfnis, die vorteilhaften Wirkungen dieser Zubereitung für weitere Indikationen im medizinischen und kosmetischen Bereich nutzbar zu machen.

### DARSTELLUNG DER ERFINDUNG

Diese Aufgaben werden von der Erfindung durch eine Hautauflage nach Anspruch 1 gelöst. Vorteilhafte Ausgestaltungen und weitere Ausführungsbeispiele sind in den abhängigen Ansprüchen beschrieben.

Weiter werden diese Aufgaben von der Erfindung durch einen Applikationsbeutel gelöst.

Eine Hautauflage zur Applikation auf der Haut oder einer Wunde und zur Abgabe von Ionen gemäss der vorliegenden Erfindung umfasst mindestens eine Lage einer festen, aktiven Substanz und mindestens eine hautzugewandte, wasser- und ionendurchlässige, hautverträgliche Durchlasslage. Die feste, aktive Substanz umfasst mindestens ein Salz eines biologisch abbaubaren Phosphates aus der Gruppe der Phosphorsäure, Metaphosphorsäure, Diphosphorsäure und die Gegenionen dieser Salze, vorzugsweise Erdalkalimetallionen, besonders bevorzugt Kalziummetaphosphat Ca(PO₃)₂, beta-Tricalziumphosphat Ca₃(PO₄)₂ und Hydroxylapathit Ca₃(PO₄)₃OH.

In einer bevorzugten Ausführungsform sind die Alkalimetallionen Natrium- und/oder Kaliumionen. Diese können jeder Form von den entsprechenden Halogeniden oder Oxiden zu der Zubereitung zugegeben werden, oder sie können auch schon als Gegenionen der biologisch abbaubaren Phosphate neben den Erdalkalimetallionen vorkommen. Besonders bevorzugt sind Natrium-Calzium-Diphosphate und Kalium-Calzium-Diphosphate.

In einer weiteren bevorzugten Ausführungsform kommt neben Kalzium ein weiteres Erdalkalimetallion, nämlich Magnesium, vor. Dies kann als Gegenion der biologisch abbaubaren Phosphate, als Halogenidsalz oder als Oxid der Zubereitung zugegeben werden.

Die erfindungsgemässe Hautauflage erlaubt es nun, dass die feste, aktive Substanz nicht mehr frei auf die Wunde aufgetragen werden muss, was bei der Applikation und der Entfernung Geschicklichkeit und Aufwand erfordert. Neu kann die feste, aktive Substanz in einer Hautauflage auf der Haut oder auf der Wunde appliziert werden. Der Vorteil ist, dass die feste, aktive Substanz bereits portioniert zur Applikation zur Verfügung steht, sofort angewendet werden kann und in toto wieder entfernbar ist. Als besonders vorteilhaft hat sich erwiesen, dass keine Partikel auf der Haut oder in der Wunde verbleiben und so die vorgeschriebene Liegedauer des Materials nicht überschritten wird.

Die Hautauflage ist ausreichend flüssigkeitsdurchlässig (vorzugsweise feinporig) und mechanisch stabil (insbesondere reissfest), so dass die einzelnen Partikel der festen, aktiven Substanz in der Hautauflage bleiben, der Austritt gelöster Ionen aber problemlos gewährleistet ist und damit die Wirkung der festen, aktiven Substanz, zum Beispiel eines Granulats aus bioaktivem Glas, trotzdem nach aussen (zur Haut oder Wunde hin) entfaltet wird.

Die Hautauflage kann zum Beispiel nach dem bestimmungsgemässen Gebrauch auf der Haut oder einer Wunde zusammen mit der verbleibenden festen, aktiven Substanz rückstandslos entfernt werden, auch wenn sie möglicherweise mehrere Tage aufgelegen hat. Auch beim Einsatz als Binde oder Windel ist gewährleistet, dass keine Partikel der aktiven Substanz auf der Haut im Intimbereich zurückbleiben.

In einer Ausführungsform ist die Hautauflage dadurch gekennzeichnet, dass die feste aktive Substanz sich aus 27,1 bis 39,9 mol% P₂O₅, 22,5 bis 36,0 mol% CaO, 14,5 bis 30,0 mol% M₂O und 5,0 bis 25 mol% MgO zusammensetzt, wobei M₂O 27,1 bis 30,0 mol% Na₂O und/oder 0 bis 12 mol% K₂O enthält.

In einer Variante umfasst die Hautauflage mindestens ein Saugelement, vorzugsweise aus Zellulose, Zellulose-Flocken, Viskose, Superabsorber oder Polyurethan-(PU)-Schaum, oder eine Kombination davon. Das Saugelement dient vorteilhafterweise dazu, dass zum Beispiel Urin oder Blut, das aus einer Wunde austritt, durch die Hautauflage aufgesogen wird und so eine Wechselwirkung mit der festen, aktiven Substanz gefördert wird. Volumen und typische Beschaffenheit eines solchen Saugelements sind dem Fachmann für die verschiedenen Einsatzbereiche bekannt.

In einer Ausführungsform weist die Hautauflage eine der Durchlasslage gegenüberliegende Barrierelage auf, welche flüssigkeitsundurchlässig ist. Die Barrierelage kann zum Beispiel dazu dienen, dass die Hautauflage Flüssigkeit, welche aus dem Körper des Trägers der Hautauflage, zum Beispiel aus einer Wunde, austritt, nicht durchlässt, sondern in der Hautauflage, vorzugsweise in der Lage mit der festen, aktiven Substanz und/oder dem Saugelement, verbleibt. Dies kann vorteilhaft sein, falls über die Hautauflage Kleider angezogen werden, welche dadurch vor der aus dem Körper des Trägers der Hautauflage austretenden Flüssigkeit geschützt werden. Besonders vorteilhaft ist die Barrierelage für Ausführungsformen, bei welchen die Hautauflage eine Wundauflage, eine Binde, eine Slipeinlage oder eine Windel ist.

In einer Ausführungsform weist die Hautauflage eine zwischen Durchlasslage und Saugelement angeordnete Verteillage, vorzugsweise ein Vlies aus Polyethersulfon (PES) oder Polyethylen (PE), auf. Die Verteillage kann zum Beispiel dazu dienen, Urin oder lokalisiert aus einer Wunde austretendes Blut im Wesentlichen gleichmässig über die Fläche in der Hautauflage, vorzugsweise in Form einer Binde oder Windel, zu verteilen und dem Saugelement zuzuführen. Dies ist insbesondere vorteilhaft, da das Saugelement dadurch im Wesentlichen gleichmässig mit dem Urin oder Blut in Kontakt kommt und so eine effizientere Ausnutzung der Saugleistung und des Rückhaltevolumens gewährleistet wird.

Vorzugsweise ist die feste, aktive Substanz zwischen der Durchlasslage und dem Saugelement angeordnet. Dies hat den Vorteil, dass zwischen der festen, aktiven Substanz und der Haut oder der Wunde die Durchlasslage angeordnet ist und dass die Flüssigkeit, welche zum Saugelement gesogen wird, die Lage mit der festen, aktiven Substanz passiert und dort die Ionen löst bevor sie das Saugelement erreicht. Die gelösten Ionen diffundieren sehr schnell zum Körper des Trägers der Hautauflage hin.

Die Hautauflage kann eine oder mehrere Kammern aufweisen. Dies hat den Vorteil, dass die lokale Behandlung aller Anteile einer Wunde - also flächendeckend - gewährleistet werden kann. Die einzelnen Kammern können sicherstellen, dass sich der Inhalt nicht schwerkraftgetrieben in einem Kanten oder Eckbereich des Beutels sammelt und grosse Anteile der Wunde ohne Wirkverbindung mit der festen, aktiven Substanz sind. Die Kammern weisen gemäss bevorzugter Ausführungsformen eine Grösse und einen Inhalt gemäss der vorgenannten besonders bevorzugten Einkammer-Hautauflagen auf.

In einer Ausführungsform enthält die Hautauflage 8 bis 12 g, besonders bevorzugt 10 g der festen aktiven Substanz.

In einer Ausführungsform liegt die feste aktive Substanz in Pulverform mit einem Korndurchmesser im Bereich von 50 bis 250 µm, besonders bevorzugt zwischen 50 und 150 µm, vor, wobei das Pulver zur besseren Handhabbarkeit vorzugsweise granuliert ist und vorzugsweise ein bioaktives Glas ist.

Die feste, aktive Substanz ist vorzugsweise dadurch hergestellt, dass die vorgenannten Oxide bei einer Temperatur von 1050 ºC bis 1550 ºC geschmolzen werden. Ein dabei erhaltenes glasartiges Material wird zweckmässigerweise durch Abschrecken zerkleinert. Es kann anschliessend gemahlen werden, so dass ein Pulver mit einem mittleren Korndurchmesser im Bereich von 50 bis 250 µm, besonders bevorzugt zwischen 50 und 150 µm gebildet wird.

Das Pulver oder das Granulat kann mit pflanzlichen, homöopathischen und/oder anderen pharmazeutischen Wirkstoffen und/oder die Wundheilung unterstützenden Stoffen und/oder Wachstumsfaktoren, Proteinen, Lokaltherapeutika zur systemischen Applikation oder Gemischen davon gemischt werden.

Die feste, aktive Substanz kann als Pulver, Granulat oder auch als Suspension vorliegen. Bei Pulvern oder Granulaten hat es sich als vorteilhaft erwiesen, diese vor der eigentlichen Behandlung der Wunde anzufeuchten. Als geeignete Flüssigkeiten dazu haben sich sterile isotonische Lösungen (wie zum Beispiel eine Ringer-Lösung) erwiesen oder Lösungen die per se eine desinfizierende Wirkung aufweisen (z.B. Lavasept ®)

Die Durchlasslage kann eine Vlieslage, ein Gewebe, ein Gestrick, einen Film oder eine Membran, vorzugsweise aus Polypropylen (PP), PE, PES oder Viskose, umfassen, wobei die Durchlasslage zumindest bereichsweise adhäsive, wundschonende Materialien wie zum Beispiel hydrophobe, vliesartige Polyolefin-Folien umfasst, die Flüssigkeitsdurchtritt und lonenaustausch gewährleisten.

Der Flüssigkeitsdurchtritt und der lonenaustausch können durch Perforationen bzw. Öffnungen oder Maschen geeigneter Grösse gewährleistet werden.

Die Durchlasslage kann zumindest bereichsweise aus Naturstoff-Geweben, - Gestricken oder -Vliesen gefertigt sein und vorzugsweise mit hautverträglichen Substanzen, wie Silicon, Paraffin, Wachs und dergleichen imprägniert sein. Weiter kann die Durchlasslage mit bekannten hydrophilen Ausrüstungen ausgerüstet oder mit bekannten hydrophilen Membranen beschichtet sein.

Die Durchlasslage kann auch einen geeigneten wasserdurchlässigen Thermoplasten wie zum Beispiel Polyethylen, insbesondere PE sehr geringer Dichte (ULDPE, ultra low density polyethylene), oder durchlässige Siliconfolie oder andere Kunststoffe, oder Verbindungen von Naturstoffen und Kunststoffen umfassen.

Die Durchlasslage kann einlagig oder mehrlagig sein, wobei die Durchlasslage aus einem einzigen Material oder aus Verbundmaterialien gefertigt ist.

Wesentlich ist, dass das Material, aus dem zumindest die dem Körper zugewandte Durchlasslage der erfindungsgemässen Hautauflage gefertigt ist, derart gewählt ist, dass es nicht mit der Wunde verklebt, verbackt oder sich sonstig mit ihr verbindet. Das Hautauflagenmaterial muss biokompatibel und als Medizinalprodukt zugelassen sein.

Als besonders bevorzugt haben sich sterilisierbare Materialien erwiesen, die zudem sicherstellen, dass die Partikel aus Lage mit der festen, aktiven Substanz - im nicht benutzten Zustand der Hautauflage - nicht nach aussen gelangen können um den Sterilisationsprozess nicht zu erschweren.

Die Hautauflage kann als Beutel, Binde, Slipeinlage, Wundauflage, Bandage, Wundpad oder Windel ausgebildet sein.

Die als Beutel ausgebildete Hautauflage kann eine flächige Grundform aufweisen und vorzugsweise kreisrund mit einem Durchmesser von 5 bis 7 cm, vorzugsweise von 6 cm, rechteckig oder quadratisch mit Kantenlängen von 5 bis 7 cm, vorzugsweise von 6 cm ausgebildet sein.

Die Hautauflage in Form eines Beutels wird im Zusammenhang mit der vorliegenden Erfindung auch als Applikationsbeutel bezeichnet.

Ein Applikationsbeutel gemäss der vorliegenden Erfindung ist mit einer Zusammensetzung, vorzugsweise einer Zubereitung in Form einer festen aktiven Substanz, befüllt. Der erfindungsgemässe Applikationsbeutel erlaubt es nun dass die Zubereitung nicht mehr frei auf die Wunde aufgetragen werden muss, was bei der Applikation und der Entfernung Geschicklichkeit und Aufwand erfordert. Die Zubereitung muss auch nicht mehr mit einem Träger appliziert werden, was ebenfalls bei der Applikation einiges Geschick erfordert. Neu kann die Zusammensetzung in einem Beutel, zum Beispiel in einem Vlies-Beutel enthaltend Wundgranulat, auf der Haut oder in der Wunde appliziert werden. Der Vorteil ist, dass die Zusammensetzung bereits portioniert zur Applikation zur Verfügung steht, sofort angewendet werden kann und in toto wieder entfernbar ist. Als besonders vorteilhaft hat sich erwiesen, dass, das keine Partikel in der Wunde verbleiben und so die vorgeschriebene Liegedauer des Materials nicht überschritten wird.

### A. Aufbau eines Applikationsbeutels

Die erfindungsgemässen Beutel beinhaltend therapeutische Zusammensetzungen zur direkten Wundbehandlung bauen sich aus mindestens zwei Wandflächen auf, die einen befüllbaren oder befüllten Innenraum definieren und umschliessen, wobei zumindest die dem Körper zugewandte Wandflächen vorzugsweise vollständig, zumindest aber grossflächig, aus einem flüssigkeitsdurchlässigen, schmiegsamen Materialabschnitt zur Anpassung an die Wundoberfläche umfasst.

Das schmiegsame oder biegsame Wandmaterial kann nach dem bestimmungsgemässen Gebrauch auf der Wunde zusammen mit dem verbleibenden Beutelinhalt rückstandslos entfernt werden. Auch wenn es möglicherweise mehrere Tage auf der Wunde aufgelegen hat. Bei der Wundversorgung mit dem erfindungsgemässen Applikationsbeutel hat es sich als vorteilhaft erwiesen ihn mit einem semiokklusiven Verband an der Wunde zu halten.

Die Applikationsbeutel gemäss der vorliegenden Erfindung sind gemäss bevorzugter Ausführungsformen aus zwei gegenüberliegenden Wänden mit deckungsgleichen Grundrissen einfacher geometrischer Formen (Kreis, Quadrat, Rechteck, Oval etc.) gefertigt.

Besonders bevorzugt sind die Beutel kreisrund mit einem Durchmesser von 5 bis 7, vorzugsweise von 6 cm. Oder rechteckig oder quadratisch mit Kantenlängen von 5 bis 7, vorzugsweise von 6 cm. Die vorgenannten Beutelgrössen enthalten bevorzugter Weise 8 bis 12, besonders bevorzugt 10 g der therapeutischen Zubereitung zur direkten Wundbehandlung.

Zur Behandlung von grösseren Wunden hat es sich als vorteilhaft erwiesen flächenmässig grössere Beutel mit mehr Inhalt herzustellen. Um sicherzustellen, dass die lokale Behandlung aller Anteile der Wunde - also flächendeckend - gewährleistet ist, werden diese Beutel vorzugsweise unterkammert. Die Kammern weisen gemäss bevorzugter Ausführungsformen eine Grösse und einen Inhalt gemäss der vorgenannten besonders bevorzugten Einkammer-Beutel auf.

Für besondere Applikationen, wie zum Beispiel auf der Nase oder im Bereich der Finger oder Zehen sind die Applikationsbeutel vorzugsweise nierenförmig oder anderweitig an die räumliche Form der Applikationsstelle vorgeformt.

Die Wandelemente werden entweder umlaufend verschweisst, verklebt oder vernäht. Wird der Applikationsbeutel aus einem schlauchförmigen Wandmaterial gefertigt, so genügt es die offenen Enden nach dem Befüllen zu verschliessen.

Gemäss weiterer Ausführungsformen werden die Beutel mit einer verschliessbaren Öffnung gefertigt, die dem Endnutzer die Befüllung erlaubt.

### B. Material und Eigenschaften der Beutelwand

Die Beutelwände können ein oder mehrlagig aus einem einzigen Material oder aus Verbundmaterialien ausgebildet sein.

Wesentlich ist, dass das Material, aus dem zumindest die dem Körper zugewandte Oberflächenlage der erfindungsgemässen Applikationsbeutel gefertigt ist, derart gewählt ist, dass es nicht mit der Wunde verklebt, verbackt oder sich sonstig mit ihr verbindet. Das Beutelmaterial muss biokompatibel und als Medizinalprodukt zugelassen sein.

Gemäss erster Ausführungsformen werden Naturstoff-Gewebe, -Gestricke oder - Vliese gewählt, die mit hautverträglichen Substanzen, wie Silicon, Paraffin, Wachs und dgl. imprägniert, mit bekannten hydrophilen Ausrüstungen ausgerüstet oder mit bekannten hydrophilen Membranen beschichtet sind.

Die wasserdurchlässigen Anteile der Beutelwand können auch aus einem geeigneten wasserdurchlässigen Thermoplasten wie zum Beispiel Polyethylen, insbesondere PE sehr geringer Dichte (ULDPE, ultra low density polyethlene), oder durchlässiger Siliconfolie oder anderen Kunststoffen, oder Verbindungen von Naturstoffen und Kunststoffen gewählt werden.

Gemäss weiterer Ausführungsformen sind die Beutel nicht einfach flächig geformt sondern in Form eines dreidimensionalen Körpers als Wundfüller.

Die in Kontakt mit der Haut des Patienten kommenden proximalen Anteile der Beutelwand sind gemäss bevorzugter Ausführungsformen aus adhäsiven, wundschonenden Materialien wie zum Beispiel hydrophoben, vliesartigen Polyolefin-Folien, wie Polyethylen- oder Polypropylen-Folie, die durch Perforationen bzw. Öffnungen oder Maschen geeigneter Grösse den Flüssigkeitsdurchtritt und lonenaustausch gewährleisten.

Als besonders bevorzugt haben sich sterilisierbare Materialien erwiesen, die zudem sicherstellen, dass die Partikel aus dem Inneren des Beutels - im nicht benutzten Zustand des Beutels - nicht nach aussen gelangen können um den Sterilisationsprozess nicht zu erschweren.

### C. Inhalt des Beutels

Wie bereits erwähnt, umfasst die Zusammensetzung zur Befüllung des Beutels vorzugsweise eine Zubereitung in Form einer festen aktiven Substanz, die im weitesten Sinne als bioaktives Glas bezeichnet werden kann. Bevorzugt umfasst das bioaktive Glas ein Salz eines biologisch abbaubaren Phosphates aus der Gruppe der Phosphorsäure, Metaphosphorsäure, Diphosphorsäure und die Gegenionen dieser Salze vorzugsweise Erdalkalimetallionen. Besonders bevorzugt sind Kalziummetaphosphat Ca(PO₃)₂, beta-Tricalziumphosphat Ca₃(PO₄), und Hydroxylapathit Ca₃(PO₄)₃OH. Diese Salze eines biologisch abbaubaren Phosphates können jeweils alleine oder in Mischungen vorkommen.

In einer weiteren bevorzugten Ausführungsform sind die Alkalimetallionen Natrium- und/oder Kaliumionen. Diese können jeder Form von den entsprechenden Halogeniden oder Oxiden zu der Zubereitung zugegeben werden, oder sie können auch schon als Gegenionen der biologisch abbaubaren Phosphate neben den Erdalkalimetallionen vorkommen. Besonders bevorzugt sind Natrium-Calzium-Diphosphate und Kalium-Calzium-Diphosphate.

In einer weiteren bevorzugten Ausführungsform kommt neben Kalzium ein weiteres Erdalkalimetallion, nämlich Magnesium, vor. Dies kann als Gegenion der biologisch abbaubaren Phosphate, als Halogenidsalz oder als Oxid der Zubereitung zugegeben werden.

Gemäss einer bevorzugten Ausführungsform setzt sich die Zubereitung aus 27,1 bis 39,9 mol% P₂O₅, 22,5 bis 36,0 mol% CaO, 14,5 bis 30,0 mol% M₂O und 5,0 bis 25 mol% MgO zusammen, wobei M₂O 27,1 bis 30,0 mol% Na₂O und/oder 0 bis 12 mol% K₂O enthält.

Die zur Befüllung des Beutels verwendete Zusammensetzung umfasst eine Zubereitung die vorzugsweise dadurch hergestellt ist, dass die vorgenannten Oxide bei einer Temperatur von 1050 ºC bis 1550 ºC geschmolzen werden. Ein dabei erhaltenes glas-artiges Material wird zweckmässigerweise durch Abschrecken zerkleinert. Es kann anschliessend gemahlen werden, so dass ein Pulver mit einem mittleren Korndurchmesser im Bereich von 50 bis 250 µm, besonders bevorzugt zwischen 50 und 150 µm gebildet wird. Das Pulver kann anschliessend zur besseren Handhabbarkeit granuliert werden. Das Pulver oder das Granulat können zur Herstellung der Zusammensetzung anschliessend mit pflanzlichen, homöopathischen und/oder anderen pharmazeutischen Wirkstoffen und/oder die Wundheilung unterstützenden Stoffen und/oder Wachstumsfaktoren, Proteinen oder dgl. gemischt werden.

Die zur Befüllung des Beutels verwendete Zusammensetzung umfasst gemäss weiterer bevorzugter Ausführungsformen pflanzliche, homöopathische und/oder andere pharmazeutische Wirkstoffen und/oder die Wundheilung unterstützenden Stoffe und/oder Wachstumsfaktoren, Proteinen, ähnlichen Lokaltherapeutika zur systemischen Applikation oder dgl. oder Gemische davon.

Alle zur Befüllung des Beutels verwendeten Zusammensetzungen liegen vorzugsweise entweder als Pulver, Granulat oder auch als Suspension vor. Bei Pulvern oder Granulaten hat es sich als vorteilhaft erwiesen diese vor der eigentlichen Behandlung der Wunde anzufeuchten. Als geeignete Flüssigkeiten dazu haben sich sterile isotonische Lösungen (wie zum Beispiel eine Ringer-Lösung) erwiesen oder Lösungen die per se eine desinfizierende Wirkung aufweisen (z.B. Lavasept ®).

### KURZE ERLÄUTERUNG DER FIGUREN

Ausführungsformen der Erfindung werden anhand der nachfolgenden Figuren und der dazugehörigen Beschreibungen näher erläutert. Es zeigen:
- Fig. 1: eine Ausführungsform der Hautauflage als Wundauflage in schematischer Querschnittdarstellung;
- Fig. 2: eine Ausführungsform der Hautauflage als Wundpad schematisch im Querschnitt;
- Fig. 3: eine Ausführungsform der Hautauflage als Binde oder Slipeinlage oder Windel schematisch im Querschnitt;
- Fig. 4: eine Ausführungsform der Hautauflage als Bandage in schematischer Querschnittdarstellung.

### DETAILLIERTE BESCHREIBUNG DER AUSFÜHRUNGSFORMEN

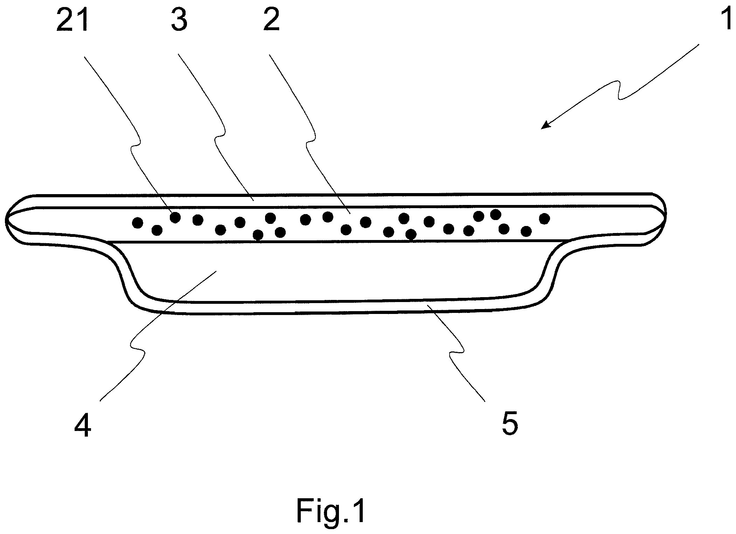

Fig. 1 zeigt eine Ausführungsform der Hautauflage als Wundauflage 1 in Querschnittdarstellung senkrecht zur Ausdehnungsfläche der Wundauflage 1. Die Wundauflage 1 umfasst eine hautzugewandte Durchlasslage 3, welche wasser- und ionendurchlässig und vorzugsweise hautverträglich ist. Die Durchlasslage 3 kann ein Vlies, eine Membran oder ein Film aus PP, PE oder PES sein. An der Durchlasslage 3 anschliessend ist eine Lage 2 einer festen, aktiven Substanz 21 angeordnet. Die feste, aktive Substanz 21 kann zum Beispiel ein Salz eines biologisch abbaubaren Phosphates aus der Gruppe der Phosphorsäure, Metaphosphorsäure, Diphosphorsäure und die Gegenionen dieser Salze, vorzugsweise Erdalkalimetallionen, besonders bevorzugt Kalziummetaphosphat Ca(PO₃)₂, beta-Tricalziumphosphat Ca₃(PO₄), und Hydroxylapathit Ca₃(PO₄)₃OH sein. Die feste, aktive Substanz 21 kann zur besseren Handhabung granuliert sein. An der Lage 2 mit der festen, aktiven Substanz 21 anschliessend ist ein Saugelement 4 angeordnet, das zum Beispiel aus Zellulose, Zellulose-Flocken, Viskose, Superabsorber oder PU-Schaum, oder einer Kombination davon bestehen kann. Am Saugelement 4 anschliessend und der Durchlasslage 3 gegenüberliegend ist eine Barrierelage 5 angeordnet, welche flüssigkeitsundurchlässig ist und somit verhindert, dass zum Beispiel Blut oder Lymphe, welche durch die Durchlasslage 3, über die Lage 2 mit der festen, aktiven Substanz 21 zum Saugelement 4 tritt, weiter über die Barrierelage 5 aus der Wundauflage 1 austritt. Somit kann die Wundauflage 1 auf eine Wunde aufgebracht werden und darüber zum Beispiel ein Kleid angezogen werden, ohne dass das Kleid vom Blut beschmutzt wird. Die Barrierelage 5 kann ein Film aus PU sein.

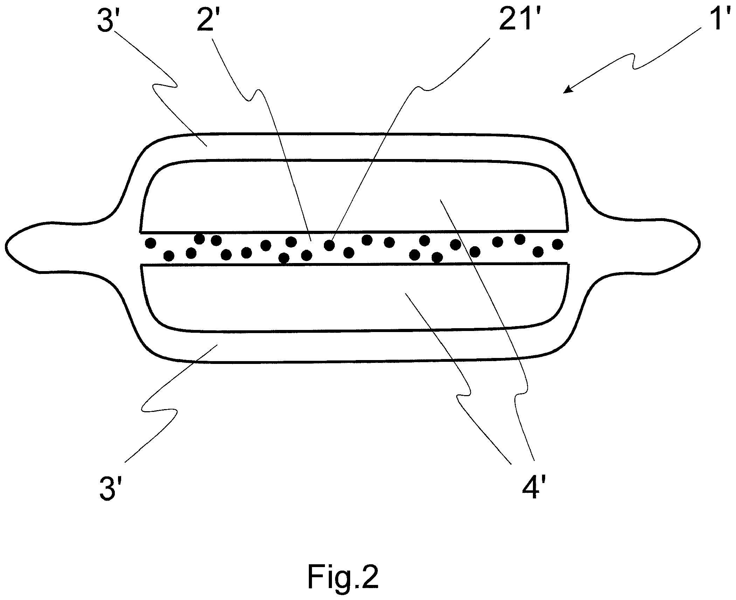

Fig. 2 zeigt eine Ausführungsform der Hautauflage als Wundpad 1' in einer Querschnittdarstellung senkrecht zur Ausdehnungsfläche des Wundpads 1'. Das Wundpad 1' umfasst eine Durchlasslage 3', welche aus Hüllvlies 3' ausgebildet ist und das ganze Wundpad 1' umhüllt. Das Hüllvlies 3' kann aus Viskose oder PES sein. Mittig ist eine Lage 2' mit einer festen, aktiven Substanz 21' angeordnet. Die feste, aktive Substanz 21' kann zum Beispiel ein Salz eines biologisch abbaubaren Phosphates aus der Gruppe der Phosphorsäure, Metaphosphorsäure, Diphosphorsäure und die Gegenionen dieser Salze, vorzugsweise Erdalkalimetallionen, besonders bevorzugt Kalziummetaphosphat Ca(PO₃)₂, beta-Tricalziumphosphat Ca₃(PO₄)₂ und Hydroxylapathit Ca₃(PO₄)₃OH sein. Zwischen der Lage 2' und der Durchlasslage 3' sind beidseits der Lage 2' zwei Saugelemente 4' angeordnet. Die Saugelemente 4' können aus Viskose oder PES sein. Durch die gezeigte symmetrische Anordnung der Saugelemente 4' und der Durchlasslage 3' kann das Wundpad 1' beidseitig angewendet werden, was bei einer schnellen Wundversorgung besonders vorteilhaft sein kann, da sichergestellt wird, dass das Wundpad 1' immer richtig auf eine Wunde aufgebracht wird.

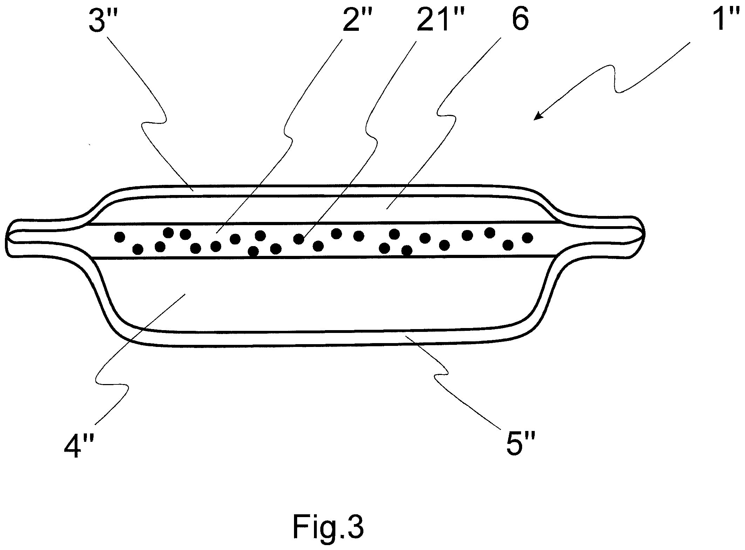

Fig. 3 zeigt eine Ausführungsform einer Hautauflage als Binde 1" in Querschnittdarstellung senkrecht zur Ausdehnungsfläche der Binde 1". Ausführungsformen der Hautauflage als Slipeinlage oder Windel haben im Wesentlichen den gleichen Aufbau wie der gezeigten Binde 1". Die Binde 1" umfasst eine körperzugewandte Durchlasslage 3", welche wasser- und ionendurchlässig und hautverträglich ist. Die Durchlasslage 3" kann ein Vlies aus PP, PE oder PES sein. An der Durchlasslage 3" anschliessend ist eine Verteillage 6 angeordnet, welche zur gleichmässigen Verteilung von Flüssigkeit über die Fläche der Binde 1" dient. Die Verteillage 6 ist vorzugsweise ein Vlies aus PES oder PE. An der Verteillage 6 anschliessend ist eine Lage 2" mit einer festen, aktiven Substanz 21" angeordnet. Die feste, aktive Substanz 21" kann zum Beispiel ein Salz eines biologisch abbaubaren Phosphates aus der Gruppe der Phosphorsäure, Metaphosphorsäure, Diphosphorsäure und die Gegenionen dieser Salze, vorzugsweise Erdalkalimetallionen, besonders bevorzugt Kalziummetaphosphat Ca(PO₃)₂, beta-Tricalziumphosphat Ca₃(PO₄)₂ und Hydroxylapathit Ca₃(PO₄)₃OH sein. An der Lage 2" anschliessend ist ein Saugelement 4" angeordnet, an welchem eine Barrierelage 5" anschliesst. Das Saugelement 4" kann aus Zellulose Fluff oder Superabsorber sein. Die Barrierelage 5" ist flüssigkeitsundurchlässig und verhindert, dass Flüssigkeit über die Barrierelage 5" aus der Binde 1" austritt. Ein solcher Aufbau wie die gezeigte Binde 1" mit der Verteillage 6 und der Barrierelage 5" ist auch besonders vorteilhaft für Slipeinlagen oder Windeln. Die Barrierelage 5" kann zum Beispiel ein PE-Film mit PP-Spinnvlies sein.

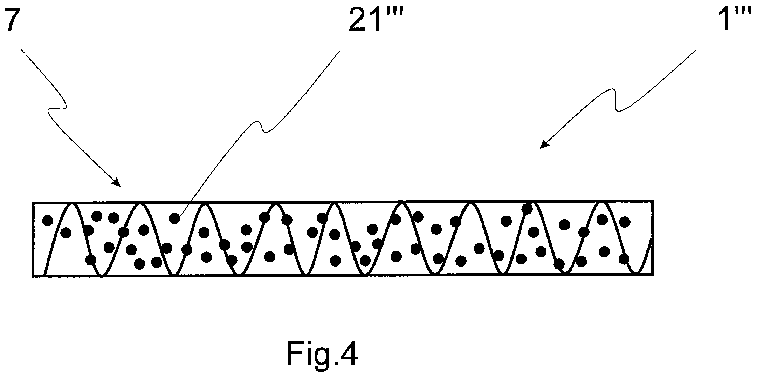

Fig. 4 zeigt eine Ausführungsform der Hautauflage als Bandage 1'" in Querschnittdarstellung senkrecht zur Ausdehnungsfläche der Bandage 1"'. Die Bandage 1"' umfasst ein Gewebe 7. Das Gewebe 7 enthält eine feste, aktive Substanz 21"', welche zum Beispiel ein Salz eines biologisch abbaubaren Phosphates aus der Gruppe der Phosphorsäure, Metaphosphorsäure, Diphosphorsäure und die Gegenionen dieser Salze, vorzugsweise Erdalkalimetallionen, besonders bevorzugt Kalziummetaphosphat Ca(PO₃)₂, beta-Tricalziumphosphat Ca₃(PO₄), und Hydroxylapathit Ca₃(PO₄)₃OH sein kann. Das Gewebe 7 kann ein Webband aus Baumwolle oder Viskose mit Polyamid sein.

## Patentansprüche

1. Hautauflage (1, 1', 1", 1"') zur Applikation auf der Haut oder einer Wunde und zur Abgabe von Ionen, die Hautauflage (1, 1', 1", 1"') umfassend mindestens eine Lage (2, 2', 2") einer festen, aktiven Substanz (21, 21', 21", 21"') und mindestens eine hautzugewandte, wasser- und ionendurchlässige, hautverträgliche Durchlasslage (3, 3', 3"), wobei die feste, aktive Substanz (21, 21', 21", 21'") mindestens ein Salz eines biologisch abbaubaren Phosphates aus der Gruppe der Phosphorsäure, Metaphosphorsäure, Diphosphorsäure und die Gegenionen dieser Salze, vorzugsweise Erdalkalimetallionen, besonders bevorzugt Kalziummetaphosphat Ca(PO₃)₂, beta-Tricalziumphosphat Ca₃(PO₄), und Hydroxylapathit Ca₃(PO₄)₃OH, umfasst.

2. Hautauflage (1, 1', 1", 1'") gemäss Anspruch 1, **dadurch gekennzeichnet, dass** die feste, aktive Substanz (21, 21', 21", 21'") sich aus 27,1 bis 39,9 mol% P₂O₅, 22,5 bis 36,0 mol% CaO, 14,5 bis 30,0 mol% M₂O und 5,0 bis 25 mol% MgO zusammensetzt, wobei M₂O 27,1 bis 30,0 mol% Na₂O und/oder 0 bis 12 mol% K₂O enthält.

3. Hautauflage (1, 1', 1", 1'") gemäss einem der vorhergehenden Ansprüche, **dadurch gekennzeichnet, dass** die Hautauflage (1, 1', 1", 1"') mindestens ein Saugelement (4, 4', 4"), vorzugsweise aus Zellulose, Zellulose-Flocken, Viskose, Superabsorber oder Polyurethan-(PU)-Schaum, oder eine Kombination davon, umfasst.

4. Hautauflage (1, 1") gemäss einem der vorhergehenden Ansprüche, **dadurch gekennzeichnet, dass** die Hautauflage (1, 1") eine der Durchlasslage (3, 3") gegenüberliegende Barrierelage (5, 5") aufweist, welche flüssigkeitsundurchlässig ist.

5. Hautauflage (1") gemäss einem der Ansprüche 3 oder 4, **dadurch gekennzeichnet, dass** die Hautauflage (1") eine zwischen Durchlasslage (3") und Saugelement (4") angeordnete Verteillage (6), vorzugsweise ein Vlies aus Polyethersulfon (PES) oder Polyethylen (PE), aufweist.

6. Hautauflage (1, 1', 1", 1"') gemäss einem der Ansprüche 3 bis 5, **dadurch gekennzeichnet, dass** die feste aktive Substanz (21, 21', 21", 21'") zwischen der Durchlasslage (3, 3', 3") und dem Saugelement (4, 4', 4") angeordnet ist.

7. Hautauflage (1, 1', 1", 1"') gemäss einem der vorhergehenden Ansprüche, **dadurch gekennzeichnet, dass** die Hautauflage (1, 1', 1", 1"') eine oder mehrere Kammern aufweist.

8. Hautauflage (1, 1', 1", 1'") gemäss einem der vorhergehenden Ansprüche, **dadurch gekennzeichnet, dass** die Hautauflage (1, 1', 1", 1"') 8 bis 12 g, besonders bevorzugt 10 g der festen aktiven Substanz (21, 21', 21", 21'") enthält.

9. Hautauflage (1, 1', 1", 1"') gemäss einem der vorhergehenden Ansprüche, **dadurch gekennzeichnet, dass** die feste aktive Substanz (21, 21', 21", 21"') in Pulverform mit einem Korndurchmesser im Bereich von 50 bis 250 µm, besonders bevorzugt zwischen 50 und 150 µm, vorliegt, wobei das Pulver zur besseren Handhabbarkeit vorzugsweise granuliert ist und vorzugsweise ein bioaktives Glas ist.

10. Hautauflage (1, 1', 1", 1'") gemäss Anspruch 9, **dadurch gekennzeichnet, dass** das Pulver oder das Granulat mit pflanzlichen, homöopathischen und/oder anderen pharmazeutischen Wirkstoffen und/oder die Wundheilung unterstützenden Stoffen und/oder Wachstumsfaktoren, Proteinen, Lokaltherapeutika zur systemischen Applikation oder Gemischen davon gemischt werden.

11. Hautauflage (1, 1', 1", 1'") gemäss einem der vorhergehenden Ansprüche, **dadurch gekennzeichnet, dass** die Durchlasslage (3, 3', 3") eine Vlieslage, ein Gewebe, ein Gestrick, einen Film oder eine Membran, vorzugsweise aus Polypropylen (PP), PE, PES oder Viskose, umfasst, wobei die Durchlasslage (3, 3', 3") zumindest bereichsweise adhäsive, wundschonende Materialien wie zum Beispiel hydrophobe, vliesartige Polyolefin-Folien umfasst, die Flüssigkeitsdurchtritt und lonenaustausch gewährleisten.

12. Hautauflage (1, 1', 1", 1'") gemäss einem der vorhergehenden Ansprüche, **dadurch gekennzeichnet, dass** die Durchlasslage (3, 3', 3") zumindest bereichsweise aus Naturstoff-Geweben, -Gestricken oder -Vliesen gefertigt ist und vorzugsweise mit hautverträglichen Substanzen, wie Silicon, Paraffin, Wachs und dergleichen imprägniert sein kann.

13. Hautauflage (1, 1', 1", 1"') gemäss einem der vorhergehenden Ansprüche, **dadurch gekennzeichnet, dass** die Durchlasslage (3, 3', 3") einlagig oder mehrlagig ist, wobei die Durchlasslage (3, 3', 3") aus einem einzigen Material oder aus Verbundmaterialien gefertigt ist.

14. Hautauflage (1, 1', 1", 1"') gemäss einem der vorhergehenden Ansprüche, **dadurch gekennzeichnet, dass** die Hautauflage (1, 1', 1", 1"') als Beutel, Binde (1"), Slipeinlage (1"), Wundauflage (1), Bandage (1'"), Wundpad (1') oder Windel (1") ausgebildet ist.

15. Hautauflage (1, 1', 1", 1"') gemäss Patentanspruch 14, **dadurch gekennzeichnet, dass** die Hautauflage (1, 1', 1", 1'") als Beutel, der eine flächige Grundform aufweist und vorzugsweise kreisrund mit einem Durchmesser von 5 bis 7 cm, vorzugsweise von 6 cm, rechteckig oder quadratisch mit Kantenlängen von 5 bis 7 cm, vorzugsweise von 6 cm ausgebildet ist.

## Claims

1. Skin contact article (1, 1', 1", 1"') for application on the skin or a wound and for delivery of ions, the skin contact article (1, 1', 1", 1"') comprising at least one ply (2, 2', 2") of a solid, active substance (21, 21', 21", 21"') and comprising at least one skin-facing, water- and ion-transmissive, skin-compatible transmission ply (3, 3', 3"), wherein the solid, active substance (21, 21', 21", 21"') comprises at least one salt of a biodegradable phosphate from the group of phosphoric acid, metaphosphoric acid, diphosphoric acid and the counter-ions of these salts, preferably alkaline earth metal ions, more preferably calcium metaphosphate Ca(PO₃)₂, beta-tricalcium phosphate Ca₃(PO₄)₂ and hydroxylapatite Ca₃(PO₄)₃OH.

2. Skin contact article (1, 1', 1", 1"') according to Claim 1, **characterized in that** the solid, active substance (21, 21', 21", 21"') is composed of 27.1 to 39.9 mol% P₂O₅, 22.5 to 36.0 mol% CaO, 14.5 to 30.0 mol% M₂O and 5.0 to 25 mol% MgO, wherein M₂O comprises 27.1 to 30.0 mol% Na₂O and/or 0 to 12 mol% K₂O.

3. Skin contact article (1, 1', 1", 1"') according to either of the preceding claims, **characterized in that** the skin contact article (1, 1', 1", 1"') comprises at least one wicking element (4, 4', 4"), preferably composed of cellulose, cellulose flocks, viscose, superabsorbent or polyurethane (PU) foam, or a combination thereof.

4. Skin contact article (1, 1") according to one of the preceding claims, **characterized in that** the skin contact article (1, 1") has a barrier ply (5, 5") which lies opposite the transmission ply (3, 3") and which is untransmissive for fluid.

5. Skin contact article (1") according to one of Claims 3 or 4, **characterized in that** the skin contact article (1") has a distribution ply (6) arranged between transmission ply (3") and wicking element (4"), preferably a fleece composed of polyethersulfone (PES) or polyethylene (PE).

6. Skin contact article (1, 1', 1", 1"') according to one of Claims 3 to 5, **characterized in that** the solid active substance (21, 21', 21", 21"') is arranged between the transmission ply (3, 3', 3") and the wicking element (4, 4', 4").

7. Skin contact article (1, 1', 1", 1"') according to one of the preceding claims, **characterized in that** the skin contact article (1, 1', 1", 1"') has one or more chambers.

8. Skin contact article (1, 1', 1", 1"') according to one of the preceding claims, **characterized in that** the skin contact article (1, 1', 1", 1"') contains 8 to 12 g, more preferably 10 g, of the solid active substance (21, 21', 21", 21'").

9. Skin contact article (1, 1', 1", 1"') according to one of the preceding claims, **characterized in that** the solid active substance (21, 21', 21", 21"') is present in powder form with a particle diameter in the range from 50 to 250 µm, more preferably between 50 and 150 µm, with the powder being preferably in granulated form for greater ease of handling and being preferably a bioactive glass.

10. Skin contact article (1, 1', 1", 1"') according to Claim 9, **characterized in that** the powder or the granules are mixed with active plant, homeopathic and/or other pharmaceutical ingredients and/or with wound healing promoters and/or growth factors, proteins, local therapeutic agents for systemic administration or mixtures thereof.

11. Skin contact article (1, 1', 1", 1"') according to any of the preceding claims, **characterized in that** the transmission ply (3, 3', 3") comprises a fleece ply, a woven fabric, a knitted fabric, a film or a membrane, preferably composed of polypropylene (PP), PE, PES or viscose, wherein the transmission ply (3, 3', 3") at least regionally comprises adhesive wound-sparing materials such as, for example, hydrophobic, fleece-like polyolefin foils which ensure fluid passage and ion exchange.

12. Skin contact article (1, 1', 1", 1"') according to one of the preceding claims, **characterized in that** the transmission ply (3, 3', 3") is made at least zonally of a natural woven, knitted or fleece substance and may preferably have been impregnated with skin-compatible substances, such as silicone, paraffin, wax and the like.

13. Skin contact article (1, 1', 1", 1"') according to one of the preceding claims, **characterized in that** the transmission ply (3, 3', 3") is in single-ply or multi-ply form, with the transmission ply (3, 3', 3") being made of a single material or of composite materials.

14. Skin contact article (1, 1', 1", 1"') according to one of the preceding claims, **characterized in that** the skin contact article (1, 1', 1", 1"') takes the form of a pouch, sanitary towel (1"), panty liner (1"), wound contact article (1), bandage (1"'), wound pad (1') or nappy (1").

15. Skin contact article (1, 1', 1", 1"') according to Claim 14, **characterized in that** the skin contact article (1, 1', 1", 1"') takes the form of a pouch which has a sheet-like basic form and is preferably circular with a diameter of 5 to 7 cm, preferably of 6 cm, rectangular or square with edge lengths of 5 to 7 cm, preferably of 6 cm.

## Revendications

1. Compresse pour la peau (1, 1', 1'', 1''') destinée à une application sur la peau ou sur une plaie et à la libération d'ions, la compresse pour la peau (1, 1', 1", 1''') comprenant au moins une couche (2, 2', 2'') d'une substance active solide (21, 21', 21'', 21''') et au moins une couche de passage (3, 3', 3") compatible avec la peau, perméable à l'eau et aux ions, orientée vers la peau, la substance active solide (21, 21', 21'', 21''') comprenant au moins un sel d'un phosphate biodégradable du groupe constitué par l'acide phosphorique, l'acide métaphosphorique, l'acide diphosphorique et les contre-ions de ces sels, de préférence les ions de métaux alcalino-terreux, de manière particulièrement préférée le métaphosphate de calcium Ca(PO₃)₂, le phosphate de tricalcium bêta Ca₃(PO₄), et l'hydroxyapatite Ca₃(PO₄)₃OH.

2. Compresse pour la peau (1, 1', 1'', 1"') selon la revendication 1, **caractérisée en ce que** la substance active solide (21, 21', 21'', 21''') se compose par 27,1 à 39,9 % en moles de P₂O₅, 22,5 à 36,0 % en moles de CaO, 14,5 à 30,0 % en moles de M₂O et 5,0 à 25 % en moles de MgO, M₂O contenant 27,1 à 30,0 % en moles de Na₂O et/ou 0 à 12 % en moles de K₂O.

3. Compresse pour la peau (1, 1', 1'', 1''') selon l'une quelconque des revendications précédentes, **caractérisée en ce que** la compresse pour la peau (1, 1', 1'', 1"') comprend au moins un élément aspirant (4, 4', 4''), de préférence en cellulose, flocons de cellulose, viscose, superabsorbant ou mousse de polyuréthane (PU), ou une combinaison de ceux-ci.

4. Compresse pour la peau (1, 1'') selon l'une quelconque des revendications précédentes, **caractérisée en ce que** la compresse pour la peau (1, 1'') comprend une couche de barrière (5, 5") opposée à la couche de passage (3, 3"), qui est imperméable aux liquides.

5. Compresse pour la peau (1") selon l'une quelconque des revendications 3 ou 4, **caractérisée en ce que** la compresse pour la peau (1") comprend une couche de distribution (6) agencée entre la couche de passage (3") et l'élément aspirant (4"), de préférence un non-tissé en polyéther-sulfone (PES) ou en polyéthylène (PE).

6. Compresse pour la peau (1, 1', 1'', 1"') selon l'une quelconque des revendications 3 à 5, **caractérisée en ce que** la substance active solide (21, 21', 21'', 21''') est agencée entre la couche de passage (3, 3', 3") et l'élément aspirant (4, 4', 4").

7. Compresse pour la peau (1, 1', 1'', 1"') selon l'une quelconque des revendications précédentes, **caractérisée en ce que** la compresse pour la peau (1, 1', 1'', 1"') comprend une ou plusieurs chambres.

8. Compresse pour la peau (1, 1', 1'', 1"') selon l'une quelconque des revendications précédentes, **caractérisée en ce que** la compresse pour la peau (1, 1', 1'', 1''') contient 8 à 12 g, de manière particulièrement préférée 10 g, de la substance active solide (21, 21', 21" , 21"') .

9. Compresse pour la peau (1, 1', 1'', 1''') selon l'une quelconque des revendications précédentes, **caractérisée en ce que** la substance active solide (21, 21', 21'', 21''') se présente sous la forme d'une poudre ayant un diamètre de particule dans la plage allant de 50 à 250 µm, de manière particulièrement préférée compris entre 50 et 150 µm, la poudre étant de préférence granulée pour une meilleure maniabilité, et étant de préférence un verre bioactif.

10. Compresse pour la peau (1, 1', 1", 1"') selon la revendication 9, **caractérisée en ce que** la poudre ou le granulat est mélangé avec des agents actifs végétaux, homéopathiques et/ou pharmaceutiques autres et/ou des substances favorisant la cicatrisation et/ou des facteurs de croissance, des protéines, des agents thérapeutiques locaux pour l'application systémique ou des mélanges de ceux-ci.

11. Compresse pour la peau (1, 1', 1", 1"') selon l'une quelconque des revendications précédentes, **caractérisée en ce que** la couche de passage (3, 3', 3") comprend une couche de non-tissé, un tissu, un tricot, un film ou une membrane, de préférence en polypropylène (PP), PE, PES ou viscose, la couche de passage (3, 3', 3") comprenant au moins en zones des matériaux adhésifs doux pour les plaies, tels que par exemple des films de polyoléfine hydrophobes de type non-tissés, qui assurent le passage des liquides et l'échange d'ions.

12. Compresse pour la peau (1, 1', 1", 1"') selon l'une quelconque des revendications précédentes, **caractérisée en ce que** la couche de passage (3, 3', 3") est fabriquée au moins en zones par des tissus, tricots ou non-tissés en matières naturelles, et peut de préférence être imprégnée avec des substances compatibles avec la peau, telles qu'une silicone, une paraffine, une cire et analogues.

13. Compresse pour la peau (1, 1', 1", 1"') selon l'une quelconque des revendications précédentes, **caractérisée en ce que** la couche de passage (3, 3', 3") est monocouche ou multicouche, la couche de passage (3, 3', 3") étant fabriquée en un matériau unique ou des matériaux composites.

14. Compresse pour la peau (1, 1', 1", 1"') selon l'une quelconque des revendications précédentes, **caractérisée en ce que** la compresse pour la peau (1, 1', 1'', 1''') est configurée sous la forme d'une poche, d'une serviette hygiénique (1"), d'un protège-slip (1"), d'un pansement (1), d'un bandage (1"'), d'une compresse pour plaies (1') ou d'une couche (1").

15. Compresse pour la peau (1, 1', 1", 1"') selon la revendication 14, **caractérisée en ce que** la compresse pour la peau (1, 1', 1", 1''') est configurée sous la forme d'une poche, qui présente une forme de base plate et de préférence circulaire avec un diamètre de 5 à 7 cm, de préférence de 6 cm, rectangulaire ou carrée avec des longueurs de bords de 5 à 7 cm, de préférence de 6 cm.
